# EUROPÄISCHE PATENTSCHRIFT

(11) **EP 3 535 410 B1**
(45) Veröffentlichungstag und Bekanntmachung des Hinweises auf die Patenterteilung: **14.12.2022**
(21) Anmeldenummer: 18702110.0
(22) Anmeldetag: 15.01.2018
(51) Int. Cl.: C12Q 1/56, G01N 33/86, C12N 9/64

(54) **UNIVERSALES KONTROLLMATERIAL FÜR GERINNUNGSFAKTORINHIBITOR-TEST**
UNIVERSAL CONTROL MATERIAL FOR COAGULATION FACTOR INHIBITOR TESTS
MATÉRIEL DE CONTRÔLE UNIVERSEL POUR TESTS D'INHIBITEUR DE FACTEUR DE COAGULATION

(30) Priorität: 16.01.2017 EP 17151547
(43) Veröffentlichungstag der Anmeldung: 11.09.2019
(73) Patentinhaber: Siemens Healthcare Diagnostics Products GmbH, 35041 Marburg (DE)
(72) Erfinder: ZANDER, Norbert, 35039 Marburg (DE)
(86) Internationale Anmeldenummer: PCT/EP2018/050811
(87) Internationale Veröffentlichungsnummer: WO 2018/130676

(56) Entgegenhaltungen:
- EP-A1- 1 752 772
- EP-A1- 2 843 416
- WO-A1-2014/207107
- DAVID L. MCGLASSON: "Using a Single Calibration Curve With the Anti-Xa Chromogenic Assay for Monitoring Heparin Anticoagulation", LABORATORY MEDICINE, Bd. 36, Nr. 5, 1. Mai 2005 (2005-05-01), Seiten 297-299, XP055381158, US ISSN: 0007-5027, DOI: 10.1309/HYCDUCQTV8YN145Y

## Beschreibung

Die vorliegende Erfindung liegt auf dem Gebiet der Gerinnungsdiagnostik und betrifft ein universales Kontrollmaterial, dessen Herstellung und dessen Verwendung zur Qualitätskontrolle von Gerinnungsfaktorinhibitor-Testen.

Das Blutgerinnungssystem (Hämostase) erfüllt die Aufgabe, einerseits eine kontinuierliche Strömung des Blutes zu gewährleisten und anderseits im Falle einer Verletzung rasch und lokal einen Wundverschluss herbeizuführen, um einen Blutverlust zu minimieren. Die Fibrinbildung, die zur Bildung der wundverschließenden Blutgerinnsel beiträgt, steht am Ende einer Kaskade enzymatischer Prozesse, wobei jedes Enzym ein weiteres Enzym durch proteolytische Prozesse aktiviert. Beispiele für solche prokoagulatorischen Enzyme sind Thrombin (Faktor IIa) und Faktor Xa. Sowohl bei Thrombin als auch bei Faktor Xa handelt es sich um Serinproteasen, die normalerweise in Form von inaktiven Proenzymen im Blut vorkommen und erst im Zuge der Gerinnungsaktivierung aktiviert werden.

In der Antikoagulationstherapie werden zunehmend neue Gerinnungsfaktorinhibitoren, insbesondere spezifische, direkte Thrombin- (Faktor IIa-) und Faktor Xa-Inhibitoren eingesetzt. Diese neuen Gerinnungshemmer haben das Potenzial, die bislang benutzten indirekten Gerinnungshemmer, vor allem Heparin und dessen Derivate, die ihre gerinnungshemmende Wirkung nur im Zusammenspiel mit Kofaktoren wie Antithrombin oder Heparin Cofaktor II entfalten, abzulösen. Für die Steuerung der Therapie und die Dosierung der Medikamente ist es daher wichtig, die Menge des therapeutischen Inhibitors zu kennen. Es ist daher notwendig, über diagnostische Methoden zu verfügen, die es ermöglichen, die Plasmakonzentrationen der therapeutischen Inhibitoren quantitativ zu bestimmen.

Im Stand der Technik sind verschiedene Testverfahren zum quantitativen Nachweis von direkten Thrombin- bzw. direkten Faktor Xa-Inhibitoren in humanen Plasmaproben bekannt.

Die Bestimmung von Thrombin- bzw. von Faktor Xa-Inhibitoren erfolgt überlicherweise mit Hilfe chromogener oder koagulometrischer Testverfahren. Bei den chromogenen Verfahren wird die Patientenprobe, die einen Thrombin- bzw. Faktor Xa-Inhibitor vermutlich enthält, mit einer sich im Überschuss befindlichen, definierten Menge des entsprechenden aktivierten Gerinnungsfaktors und einem chromogenen Substrat für den aktivierten Gerinnungsfaktor vermischt, und die im Reaktionsansatz verbleibende Aktivität des Gerinnungsfaktors wird photometrisch gemessen. Bei den etablierten, auch kommerziell erhältlichen chromogenen Testen werden insbesondere die Chromophore para-Nitroanilin (pNA) und 5-Amino-2-Nitro-Benzoesäure (ANBA), welche ein Absorptionsmaximum bei 405 nm aufweisen, verwendet. Die entstehende gelbe Farbe wird in der Regel photometrisch bestimmt. Je höher die Konzentration des therapeutischen Inhibitors in der Patientenprobe ist, desto mehr wird die Aktivität des zugegebenen Gerinnungsfaktors gehemmt und desto weniger Substrat wird gespalten. Bei der Bestimmung von Inhibitoren verhält sich die Farbkonzentration im Testansatz umgekehrt proportional zur Inhibitorkonzentration in der Probe. Beispielsweise in EP-B1-0034320 oder in EP-A2-0004271 sind derartige Thrombin- bzw. Faktor Xa-basierte, chromogene Testverfahren beschrieben. Mit Hilfe dieser Teste können therapeutische Inhibitoren, die die Aktivität von Blutgerinnungsfaktoren inhibieren, in Patientenproben quantitativ bestimmt werden. In der WO-A1-2012069139 ist ein Verfahren zur Bestimmung von Faktor Xa-Inhibitoren und in der WO-A1-2012175183 ein Verfahren zur Bestimmung von Thrombininhibitoren in Serum- und Urinproben beschrieben.

Eine zunehmende Zahl von direkten Thrombin- bzw. von Faktor Xa-Inhibitoren ist bereits für die therapeutische Anwendung zugelassen oder befindet sich in der Zulassung. Beispiele dafür sind die Substanzen Dabigatran (PRADAXA^{®}), Argatroban (ARGATROBAN^{®}), Rivaroxaban (XARELTO^{®}), Apixaban (ELIQUIS^{®}) und Edoxaban (LIXIANA^{®}).

In der Labordiagnostik spielt die Qualitätssicherung, die sicherstellen soll, dass ein Analyseergebnis, das mit einem gegebenen Testsystem für eine Patientenprobe ermittelt wurde, präzise und richtig ist, eine wichtige Rolle. Ein allgemein praktiziertes Vorgehen besteht darin, dass man zwischen den Messungen von Patientenproben stichprobenartig und mit demselben Testsystem (Reagenzien, Analysegerät, etc.) sogenannte Kontrollproben misst, denen vom Hersteller Zielwerte für den zu bestimmenden Analyten zugewiesen sind. Entspricht das Ergebnis einer solchen Kontrollmessung dem Zielwert beziehungsweise liegt es innerhalb eines vorgegebenen Toleranzbereichs um den Zielwert, kann davon ausgegangen werden, dass auch die mit dem angewandten Testsystem für die Patientenproben ermittelten Ergebnisse frei sind von systematischen Fehlern und damit richtig und präzise sind.

Es sind verschiedene Teste für therapeutische Gerinnungsfaktor-Inhibitoren, insbesondere für Thrombin- und Faktor Xa-Inhibitoren auf dem Markt erhältlich. Derartige Teste umfassen die notwendigen Reagenzien zur quantitativen Messung der Inhibitoraktivität in einer Probe, also üblicherweise ein Thrombin- oder Faktor Xa-Reagenz und ein entsprechendes Peptidsubstrat-Reagenz sowie für den zu bestimmenden Inhibitor spezifische Kalibrator- und Kontrollmaterialien, wobei die Kalibrator- und Kontrollmaterialien definierte Mengen des zu bestimmenden Inhibitors enthalten. Beispielsweise umfasst ein Testsystem für den Thrombininhibitor Dabigatran ein Thrombin-Reagenz, ein für Thrombin spezifisches Peptidsubstrat-Reagenz und Kalibrator- und Kontrollmaterialien, die definierte Mengen Dabigatran enthalten. Während das Thrombin-Reagenz und das für Thrombin spezifische Peptidsubstrat-Reagenz auch in einem Test zur Bestimmung eines anderen Thrombininhibitors verwendet werden können, müssen die Kalibrator- und Kontrollmaterialien jedoch jeweils den spezifischen Inhibitor enthalten.

Problematisch ist die große und weiter zunehmende Anzahl therapeutischer Gerinnungsfaktor-Inhibitoren, für deren Bestimmung jeweils spezifische Kalibrator- und Kontrollmaterialien zur Verfügung gestellt werden müssen. Üblicherweise wird als Kontroll- bzw. Kalibratormaterial normales Humanplasma verwendet, dem eine definierte Menge des jeweiligen Inhibitors hinzugefügt ist. Die Herstellung spezifischer Kalibrator- und Kontrollmaterialien für jeden einzelnen therapeutischen Inhibitor bedeutet für die Hersteller der Teste einen hohen Entwicklungsaufwand.

Auch für den Anwender ist die Vielzahl der benötigten Kontroll- und Kalibratormaterialien problematisch, da er die vielen verschiedenen Kontroll- und Kalibratormaterialien anschaffen, vorrätig halten und handhaben muss. Insbesondere bei der Verwendung von automatischen Analysegeräten, die nur eine beschränkte Kapazität für die Aufnahme verschiedener Kontroll- und Kalibratormaterialien aufweisen, ist es praktisch nicht möglich, die für alle therapeutisch angewendeten Thrombin- und Faktor Xa-Inhibitoren spezifischen Kontroll- und Kalibratormaterialien im Gerät parallel zu platzieren. Ein weiterer Nachteil ist, dass die Haltbarkeit von Plasmakontrollen und -kalibratoren beschränkt ist, sodass Kontrollen und Kalibratoren für Teste, die weniger häufig durchgeführt werden, nicht vollständig aufgebraucht werden können, bevor sie wegen Ablaufs des maximalen Haltbarkeit unverbraucht entsorgt werden müssen.

Es ist daher wünschenswert, die Anzahl der notwendigen Kontroll- und Kalibratormaterialien zu reduzieren, um die vorgenannten Nachteile zu beseitigen und um die automatische Abarbeitung der verschiedenen spezifischen Teste auf einem Analysegerät sicherzustellen.

Der vorliegenden Erfindung lag daher die Aufgabe zugrunde, die geschilderten Probleme im Zusammenhang mit der Vielzahl der erforderlichen Plasmakontrollen und -kalibratoren zu beheben.

In der EP-A1-2843416 werden Kontroll-und Kalibratorplasmen beschrieben, die zur Verwendung in einem ersten Test zur Bestimmung eines ersten spezifischen Thrombininhibitors und zur Verwendung in einem zweiten Test zur Bestimmung eines ersten spezifischen Faktor Xa-Inhibitors vorgesehen sind und die den jeweiligen ersten spezifischen Thrombininhibitor und den jeweiligen ersten spezifischen Faktor Xa-Inhibitor enthalten, z.B. Dabigatran und Rivaroxaban. Auf diese Weise kann die Anzahl der notwendigen Kalibrator- und Kontrollmaterialien um 50 % verringert werden.

In der WO 2014/207107 A1 wird ein universelles Kontrollmaterial bestehend aus einer Kalibratorprobe beschrieben, in der eine definierte Menge eines therapeutischen Gerinnungsfaktorinhibitors gelöst ist (UFH, LMWH, Rivaroxaban oder Orgaran), dem ein erster Zielwert für die Konzentration des Gerinnungsfaktorinhibitors zugeordnet ist und die sich zur Verwendung in einem Verfahren zur Kontrolle der Richtigkeit von Verfahren zur quantitativen Bestimmung von therapeutischen Gerinnungsfaktorinhibitoren eignet.

Die vorliegende Erfindung löst die oben geschilderten Probleme durch die Bereitstellung eines Kontrollmaterials, das anstelle eines oder mehrerer therapeutischer Inhibitoren eine definierte Menge mindestens eines unspezifischen Serinproteaseinhibitors ausgewählt aus der Gruppe Benzamidin, Phenylmethylsulfonylfluorid und 4-(2-Aminoethyl)benzensulfonylfluorid in einer wässrigen Pufferlösung enthält und dem mindestens ein Zielwert für die Konzentration mindestens eines therapeutischen Gerinnungsfaktorinhibitors zugewiesen ist. Dies hat den Vorteil, dass für alle Teste zur quantitativen Bestimmung von therapeutischen Inhibitoren von Gerinnungsfaktoren aus der Gruppe der Serinproteasen ein einziges (universales) Kontrollmaterial verwendet werden kann.

Unter dem Begriff "Gerinnungsfaktor" ist jede plasmatische Serinprotease zu verstehen, die eine prokoagulatorische (gerinnungsfördernde) Funktion im Blutgerinnungssystem eines Säugetiers, bevorzugt des Menschen, hat. Prokoagulatorische Gerinnungsfaktoren sind in der aktivierten Form beispielsweise Faktor IIa (Thrombin), Faktor VIIa, Faktor IXa, Faktor Xa, Faktor XIa und Faktor XIIa. Im Zusammenhang mit der vorliegenden Erfindung ist, sofern nichts anderes erwähnt ist, mit einem "Gerinnungsfaktor" dessen aktive Form und nicht dessen inaktive Vorläuferform gemeint.

Die Haupttargets einer Antikoagulationsstherapie sind derzeit die Gerinnungsfaktoren Thrombin und Faktor Xa. Deshalb werden diese beiden Gerinnungsfaktoren und ihre Inhibitoren im Zusammenhang mit der vorliegenden Erfindung immer wieder beispielhaft erwähnt. Es ist jedoch durchaus möglich, dass in Zukunft alternative Therapieansätze entwickelt werden, die auf der Hemmung anderer prokoagulatorischer Gerinnungsfaktoren, wie beispielsweise Faktor VIIa, Faktor IXa, Faktor XIa oder Faktor XIIa, beruhen. Auch in diesen Fällen ist das erfindungsgemäße Vorgehen anwendbar.

Der Begriff "therapeutischer Inhibitor eines Gerinnungsfaktors" bezeichnet eine therapeutisch wirksame Substanz, die natürlicherweise nicht im menschlichen Körper vorkommt und die die proteolytische Aktivität eines einzigen Gerinnungsfaktors, also spezifisch, entweder durch direkte Bindung an den Gerinnungsfaktor oder durch die Interaktion mit physiologischen Kofaktoren, wie z.B. Antithrombin oder Heparin Cofaktor II, indirekt reduziert. Der Begriff "therapeutischer Inhibitor" umfasst explizit nicht gerinnungshemmende Proteine oder Proteinkomplexe, die natürlicherweise im menschlichen Körper vorkommen, wie z.B. Antithrombin oder Heparin Cofaktor II.

Therapeutische Inhibitoren inhibieren einen einzigen spezifischen Gerinnungsfaktor, im Allgemeinen entweder Thrombin oder Faktor Xa.

Therapeutische Gerinnungsfaktorinhibitoren aus der Gruppe der spezifischen Thrombininhibitoren sind beispielsweise Hirudin, Orgaran, Argatroban, Dabigatran, Melagatran, Ximelagatran, Bivalirudin, Lepirudin, MCC-977, SSR-182289, TGN-255, TGN-167, ARC-183 und Odiparcil.

Therapeutische Gerinnungsfaktorinhibitoren aus der Gruppe der spezifischen Faktor Xa-Inhibitoren sind beispielsweise Apixaban, Rivaroxaban, Edoxaban, Fondaparinux, Otamixaban, LY 517717, YM 153, DU-176b, DX-9065a und KFA-1982.

Unter dem Begriff "unspezifischer Serinproteaseinhibitor" ist ein Inhibitor zu verstehen, der -im Gegensatz zu den spezifischen therapeutischen Gerinnungsfaktorinhibitoreneine Vielzahl (mindestens zwei) von Gerinnungsfaktoren aus der Gruppe der Serinproteasen zu binden vermag und der durch diese Bindung die proteolytische Aktivität mehrerer Gerinnungsfaktoren reduziert. Typischerweise inhibieren diese unspezifischen Serinproteaseinhibitoren nicht nur Gerinnungsfaktoren aus der Gruppe der Serinproteasen, sondern weitere Vertreter dieser Unterfamilie der Peptidasen. In Bezug auf die vorliegende Erfindung ist jeder Serinproteaseinhibitor geeignet, der mindestens die Gerinnungsfaktoren zu inhibieren vermag, welche von den therapeutischen Inhibitoren inhibiert werden, die quantitativ bestimmt werden sollen. Bekannte unspezifische Serinproteaseinhibitoren, die eine Vielzahl von Serinproteasen, darunter auch Thrombin und Faktor Xa, zu inhibieren vermögen, sind z.B. Benzamidin, Phenylmethylsulfonylfluorid (PMSF), und 4-(2-Aminoethyl)benzensulfonylfluorid (AEBSF, auch bekannt unter dem Handelsnamen Pefabloc SC^{®}).

Gegenstand der vorliegenden Erfindung ist also ein Verfahren zur Herstellung eines Kontrollmaterials, das frei ist von therapeutischen Inhibitoren von Gerinnungsfaktoren aus der Gruppe der Serinproteasen und das zur Verwendung in einem Verfahren zur Kontrolle der Richtigkeit von Verfahren zur quantitativen Bestimmung von therapeutischen Inhibitoren von Gerinnungsfaktoren aus der Gruppe der Serinproteasen geeignet ist, das Verfahren zur Herstellung des Kontrollmaterials umfassend die Schritte:
(a) Lösen einer definierten Menge mindestens eines unspezifischen Serinproteaseinhibitors ausgewählt aus der Gruppe Benzamidin, Phenylmethylsulfonylfluorid und 4-(2-Aminoethyl)benzensulfonylfluorid in einer wässrigen Pufferlösung in einer wässrigen Pufferlösung, und
(b) Zuweisen eines ersten Zielwertes für die Konzentration eines ersten therapeutischen Gerinnungsfaktorinhibitors,
   wobei das Zuweisen des ersten Zielwertes für die Konzentration des ersten therapeutischen Gerinnungsfaktorinhibitors wie folgt durchgeführt wird:
      i) Vermischen einer ersten Teilmenge der in Schritt a) hergestellten Lösung mit einer definierten Menge eines ersten Gerinnungsfaktors und mit einem Substrat mit Spezifität für diesen Gerinnungsfaktor zu einem ersten Reaktionsansatz,
      ii) Messen der Menge eines Spaltprodukts des Substrats in dem ersten Reaktionsansatz,
      iii) Vergleichen der in dem ersten Reaktionsansatz gemessenen Menge des Spaltprodukts mit vorbestimmten Kalibrationswerten, die die in dem ersten Reaktionsansatz gemessene Menge des Spaltprodukts einer bekannten Konzentration des ersten therapeutischen Gerinnungsfaktorinhibitors zuordnen, und Ermitteln der zu der gemessenen Menge des Spaltprodukts dazugehörigen Konzentration des ersten therapeutischen Gerinnungsfaktorinhibitors, und
      iv) Festlegen der so ermittelten Konzentration des ersten therapeutischen Gerinnungsfaktorinhibitors als ersten Zielwert des Kontrollmaterials,
   wobei der erste therapeutische Gerinnungsfaktorinhibitor spezifisch den ersten Gerinnungsfaktor inhibiert und wobei der unspezifische Serinproteaseinhibitor den ersten Gerinnungsfaktor und mindestens einen weiteren Gerinnungsfaktor inhibiert.

Ein erfindungsgemäßes Kontrollmaterial ist immer frei von therapeutischen Gerinnungsfaktorinhibitoren. Es ist weiterhin vorzugsweise frei von Plasma, enthält also kein humanes und/oder tierisches Plasma. Vorzugsweise besteht ein erfindungsgemäßes Kontrollmaterial aus einer wässrigen Pufferlösung und dem darin gelösten unspezifischen Serinproteaseinhibitor. Anstelle eines unspezifischen Serinproteaseinhibitors kann auch eine Kombination von zwei oder mehr unspezifischen Serinproteaseinhibitoren in dem Kontrollmaterial enthalten sein. Geeignete wässrige Pufferlösungen sind beispielsweise phosphatgepufferte isotonische Kochsalzlösung (z.B. 0,05 M Natriumdihydrogenphosphat, 0,9 % NaCl, pH-Wert 6,9 bis 7,3), imidazolgepufferte isotonische Kochsalzlösung (z.B. 0,1 M Imidazol, 0,9 % NaCl, pH-Wert 6,9 bis 7,3), glycingepufferte isotonische Kochsalzlösung (z.B. 0,1 M Glycin, 0,9 % NaCl, pH-Wert 8,0 bis 8,2), trisgepufferte isotonische Kochsalzlösung (z.B. 0,1 M TRIS, 0,9 % NaCl, pH-Wert 6,9 bis 7,3) oder HEPES-Pufferlösung (z.B. 50 mM HEPES, pH-Wert 6,9 bis 7,4).

Gegebenenfalls kann ein Kontrollmaterial zusätzlich Antioxidanzien, Konservierungsmittel und/oder bovines Serumalbumin (BSA) enthalten.

Vorzugsweise enthält ein erfindungsgemäßes Kontrollmaterial nach dem Lösen eines unspezifischen Serinproteaseinhibitors in der Puffermatrix Benzamidin in einer Konzentration von 0.1 mM - 500 mM oder Phenylmethylsulfonylfluorid (PMSF) in einer Konzentration von 0.1 mM - 500 mM oder 4-(2-Aminoethyl)benzensulfonylfluorid (AEBSF) in einer Konzentration von 0.1 mM - 500 mM.

Im Zusammenhang mit der Herstellung eines erfindungsgemäßen Kontrollmaterials wird der durch Lösen einer definierten Menge des unspezifischen Serinproteaseinhibitors in einer wässrigen Pufferlösung bereitgestellten Lösung (die Kontrolllösung) mindestens ein erster Zielwert für die Konzentration eines ersten therapeutischen Gerinnungsfaktorinhibitors zugewiesen.

Dazu wird die Kontrolllösung mit einem solchen Testsystem analysiert wie eine später zu analysierende Patientenprobe, d.h. eine Teilmenge der Kontrolllösung wird mit entsprechenden Reagenzien vermischt, die einen ersten Gerinnungsfaktor und ein Substrat mit Spezifität für diesen Gerinnungsfaktor enthalten, und es wird die Menge eines Spaltprodukts in dem so erhaltenen Reaktionsansatz gemessen. Die gemessene Menge des Spaltprodukts wird dann mit vorbestimmten Kalibrationswerten verglichen.

Die Kalibrationswerte werden in herkömmlicher Weise vorab bestimmt, d.h. es werden mindestens zwei Kalibratormaterialien, üblicherweise humanes Normalplasma, das definierte Mengen des therapeutischen Gerinnungsfaktorinhibitors enthält, in dem zu kalibrierenden Testsystem analysiert, die erhaltenen Messwerte werden den bekannten Konzentrationen des therapeutischen Gerinnungsfaktorinhibitors zugeordnet, und es wird eine Kalibrationskurve erstellt, an der anhand der Messwerte unbekannter Proben deren Konzentration abgelesen werden kann.

Die sich anhand eines solchen Vergleichs ergebende Konzentration des therapeutischen Gerinnungsfaktorinhibitors der Kontrolllösung wird dieser dann als Zielwert zugewiesen.

Unter dem Begriff "Zielwert" ist eine Konzentrationsangabe für einen therapeutischen Gerinnungsfaktorinhibitor zu verstehen (z.B. U/mL, mg/mL etc.), die -wenn dieser therapeutische Gerinnungsfaktorinhibitor in diesem Kontrollmaterial in einem kalibrierten Testsystem quantitativ bestimmt wird- wiedergefunden werden soll. Der Begriff "Zielwert" umfasst explizit auch einen Zielwertbereich, der geringfügige, tolerable Abweichungen oberhalb und/oder unterhalb eines einzelnen Wertes umfasst.

Das Besondere an der vorliegenden Erfindung ist, dass einem erfindungsgemäß hergestellten Kontrollmaterial auch eine Vielzahl von Zielwerten (zwei, drei, vier, fünf, sechs oder mehr) für eine Vielzahl verschiedener therapeutischer Gerinnungsfaktorinhibitoren (zwei, drei, vier fünf, sechs oder mehr) zugewiesen werden kann. Die verschiedenen therapeutischen Gerinnungsfaktorinhibitoren können entweder für denselben Gerinnungsfaktor spezifisch sein (zum Beispiel mehrere unterschiedliche Thrombininhibitoren), oder sie können für verschiedene Gerinnungsfaktoren spezifisch sein (zum Beispiel ein oder mehrere Thrombininhibitoren und ein oder mehrere Faktor Xa-Inhibitoren).

Eine bevorzugte Ausführungsform des erfindungsgemäßen Verfahrens zur Herstellung eines Kontrollmaterials umfasst daher zusätzlich folgende Schritte:
(c) Zuweisen eines zweiten Zielwertes für die Konzentration eines zweiten therapeutischen Gerinnungsfaktorinhibitors,
wobei das Zuweisen des zweiten Zielwertes für die Konzentration des zweiten therapeutischen Gerinnungsfaktorinhibitors wie folgt durchgeführt wird:
   i) Vermischen einer zweiten Teilmenge der in Schritt a) hergestellten Lösung mit einer definierten Menge eines zweiten Gerinnungsfaktors und mit einem Substrat mit Spezifität für diesen Gerinnungsfaktor zu einem zweiten Reaktionsansatz,
   ii) Messen der Menge eines Spaltprodukts des Substrats in dem zweiten Reaktionsansatz,
   iii) Vergleichen der in dem zweiten Reaktionsansatz gemessenen Menge des Spaltprodukts mit vorbestimmten Kalibrationswerten, die die in dem zweiten Reaktionsansatz gemessene Menge des Spaltprodukts einer bekannten Konzentration des zweiten therapeutischen Gerinnungsfaktorinhibitors zuordnen, und Ermitteln der zu der gemessenen Menge des Spaltprodukts dazugehörigen Konzentration des zweiten therapeutischen Gerinnungsfaktorinhibitors, und
   iv) Festlegen der so ermittelten Konzentration des zweiten therapeutischen Gerinnungsfaktorinhibitors als zweiten Zielwert des Kontrollmaterials,
wobei der zweite therapeutische Gerinnungsfaktorinhibitor spezifisch den zweiten Gerinnungsfaktor inhibiert.

Das Zuweisen weiterer Zielwerte für die Konzentrationen weiterer (dritter, vierter, fünfter, sechster etc.) therapeutischer Gerinnungsfaktorinhibitoren kann in analoger Weise erfolgen.

Auf diese Weise ist ein universales Kontrollmaterial für eine Vielzahl von Gerinnungsfaktorinhibitor-Testen (zwei, drei, vier fünf, sechs oder mehr) erhältlich.

Besonders bevorzugt ist die Herstellung eines Kontrollmaterials, das zur Verwendung in einem Verfahren zur Kontrolle von einem ersten Verfahren zur quantitativen Bestimmung eines ersten Thrombininhibitors und von einem zweiten Verfahren zur quantitativen Bestimmung eines ersten Faktor Xa-Inhibitors und ggf. von weiterer Verfahren zur quantitativen Bestimmung weiterer Gerinnungsfaktorinhibitoren geeignet ist. Dazu werden dem Kontrollmaterial, wie oben beschrieben, ein erster Zielwert für die Konzentration des ersten Thrombininhibitors, z.B. Dabigatran, und ein zweiter Zielwert für die Konzentration des ersten Faktor Xa-Inhibitors, z.B. Rivaroxaban, und ggf. weitere Zielwerte zugewiesen.

Ein weiterer Gegenstand der vorliegenden Erfindung ist die Verwendung eines erfindungsgemäß hergestellten Kontrollmaterials in einem Verfahren zur Kontrolle der Richtigkeit eines Verfahrens zur quantitativen Bestimmung eines ersten therapeutischen Inhibitors eines Gerinnungsfaktors aus der Gruppe der Serinproteasen in einer Probe, wobei der erste therapeutische Gerinnungsfaktorinhibitor spezifisch einen ersten Gerinnungsfaktor inhibiert.

Eine bevorzugte Verwendung eines erfindungsgemäß hergestellten Kontrollmaterials bezieht sich auf die Verwendung in einem ersten Verfahren zur Kontrolle der Richtigkeit eines Verfahrens zur quantitativen Bestimmung des ersten therapeutischen Inhibitors eines Gerinnungsfaktors aus der Gruppe der Serinproteasen in einer Probe und/oder in einem zweiten Verfahren zur Kontrolle der Richtigkeit eines Verfahrens zur quantitativen Bestimmung des zweiten therapeutischen Inhibitors eines Gerinnungsfaktors aus der Gruppe der Serinproteasen in einer Probe. Dies setzt voraus, dass dem Kontrollmaterial sowohl ein erster Zielwert für die Konzentration eines ersten therapeutischen Gerinnungsfaktorinhibitors und ein zweiter Zielwert für die Konzentration eines zweiten therapeutischen Gerinnungsfaktorinhibitors zugewiesen sind. Wie bereits oben erläutert, kann ein erfindungsgemäß hergestelltes Kontrollmaterial, dem noch weitere Zielwerte für die Konzentrationen weiterer (dritter, vierter, fünfter, sechster etc.) therapeutischer Gerinnungsfaktorinhibitoren zugewiesen sind, auch in weiteren Verfahren zur Kontrolle der Richtigkeit von Verfahren zur quantitativen Bestimmung der weiteren therapeutischen Inhibitoren (dritter, vierter, fünfter, sechster etc.) von Gerinnungsfaktoren aus der Gruppe der Serinproteasen in einer Probe verwendet werden.

Ein Verfahren zur Kontrolle der Richtigkeit eines Verfahrens zur quantitativen Bestimmung eines therapeutischen Inhibitors eines Gerinnungsfaktors aus der Gruppe der Serinproteasen umfasst die Analyse des Kontrollmaterials in demselben Testsystem (hinsichtlich der Analysemethode und Messsystem), mit dem auch Patientenproben analysiert werden.

Bei der Kontrolle der Richtigkeit eines analytischen Verfahrens handelt es sich um ein Verfahren zur Qualitätssicherung, das sicherstellen soll, dass die Ergebnisse eines in einem Labor angewandten analytischen Verfahrens zuverlässig sind und nicht etwa aufgrund eines systematischen Fehlers zu Fehlbeurteilungen führen könnten.

Dazu wird geprüft, ob das mit einer Kontrollprobe ermittelte Analyseergebnis dem der Kontrollprobe zugewiesenen Zielwert (oder Zielwertebereich) entspricht. Stellt sich heraus, dass das mit einer Kontrollprobe ermittelte Analyseergebnis dem Zielwert der Kontrollprobe entspricht, wird festgestellt, dass das analytische Verfahren zuverlässig funktioniert und richtige Ergebnisse liefert. Stellt sich heraus, dass das mit einer Kontrollprobe ermittelte Analyseergebnis dem Zielwert der Kontrollprobe nicht entspricht, wird festgestellt, dass das analytische Verfahren nicht zuverlässig funktioniert und dass die Ergebnisse dieses analytischen Verfahrens wegen des Risikos von Fehlbeurteilungen nicht zur Beurteilung von Patientenproben verwendet werden sollten.

Ein weiterer Gegenstand der vorliegenden Erfindung ist also ein Verfahren zur Kontrolle der Richtigkeit eines Verfahrens zur quantitativen Bestimmung eines therapeutischen Inhibitors eines ersten Gerinnungsfaktors aus der Gruppe der Serinproteasen in einer Patientenprobe, wobei der erste therapeutische Gerinnungsfaktorinhibitor spezifisch den ersten Gerinnungsfaktor inhibiert. Das Verfahren zur quantitativen Bestimmung des therapeutischen Gerinnungsfaktorinhibitors umfasst folgende Schritte:
a. Vermischen der Patientenprobe mit einer definierten Menge des ersten Gerinnungsfaktors und mit einem Substrat mit Spezifität für diesen Gerinnungsfaktor zu einem Reaktionsansatz,
b. Messen der Menge eines Spaltprodukts des Substrates in dem Reaktionsansatz,
c. Vermischen von mindestens zwei Kalibratorproben, die jeweils eine definierte Menge des therapeutischen Gerinnungsfaktorinhibitors enthalten, mit derselben definierten Menge des ersten Gerinnungsfaktors und mit demselben Substrat mit Spezifität für diesen Gerinnungsfaktor zu jeweils einem Kalibratorreaktionsansatz,
d. Messen der Menge desselben Spaltprodukts des Substrates in den Kalibratorreaktionsansätzen und Erstellen von Kalibrationswerten, die die in den Kalibratorreaktionsansätzen gemessene Menge des Spaltprodukts einer bekannten Konzentration des ersten therapeutischen Gerinnungsfaktorinhibitors zuordnen, und Ermitteln der zu der gemessenen Menge des Spaltprodukts dazugehörigen Konzentration des ersten therapeutischen Gerinnungsfaktorinhibitors in der Patientenprobe, und
e. Bestimmen der Menge des in der Patientenprobe enthaltenen therapeutischen Gerinnungsfaktorinhibitors durch Vergleichen der in dem Reaktionsansatz gemessenen Menge des Spaltprodukts mit den Kalibrationswerten.

Zur Kontrolle der Richtigkeit des Verfahrens zur quantitativen Bestimmung des therapeutischen Gerinnungsfaktorinhibitors werden zusätzliche folgende Schritte durchgeführt:
i) Vermischen einer Kontrollprobe bestehend aus einer Teilmenge eines erfindungsgemäß hergestellten Kontrollmaterials mit derselben definierten Menge des ersten Gerinnungsfaktors und mit demselben Substrat mit Spezifität für diesen Gerinnungsfaktor zu einem Kontrollreaktionsansatz,
ii) Messen der Menge desselben Spaltprodukts des Substrates in dem Kontrollreaktionsansatz,
iii) Vergleichen der in dem Kontrollreaktionsansatz gemessenen Menge des Spaltprodukts mit den Kalibrationswerten und Ermitteln der Konzentration des ersten therapeutischen Gerinnungsfaktorinhibitors in der Kontrollprobe, und
   A. Feststellen, dass die Richtigkeit des Verfahrens zur quantitativen Bestimmung des therapeutischen Gerinnungsfaktorinhibitors in der Patientenprobe gegeben ist, wenn die in Schritt iii) in der Kontrollprobe ermittelte Konzentration des ersten therapeutischen Gerinnungsfaktorinhibitors dem ersten Zielwert für die Konzentration eines ersten therapeutischen Gerinnungsfaktorinhibitors der Kontrollprobe entspricht oder
   B. Feststellen, dass die Richtigkeit des Verfahrens zur quantitativen Bestimmung des therapeutischen Gerinnungsfaktorinhibitors nicht gegeben ist, wenn die in Schritt iii) in der Kontrollprobe ermittelte Konzentration des ersten therapeutischen Gerinnungsfaktorinhibitors von dem ersten Zielwert für die Konzentration eines ersten therapeutischen Gerinnungsfaktorinhibitors der Kontrollprobe abweicht.

Wenn im Zusammenhang mit der vorliegenden Erfindung die Rede ist von einem Verfahren zur quantitativen Bestimmung eines therapeutischen Gerinnungsfaktorinhibitors umfassend die Schritte:
a. Vermischen einer Probe mit einer definierten Menge eines Gerinnungsfaktors und mit einem Substrat mit Spezifität für diesen Gerinnungsfaktor zu einem Reaktionsansatz, und
b. Messen der Menge eines Spaltprodukts des Substrates in dem Reaktionsansatz,
handelt es sich im Wesentlichen um aus dem Stand der Technik bekannte Gerinnungsfaktoraktivitätsteste, die die Messung der Hemmung der Gerinnungsfaktoraktivität ermöglichen.

Unter einer "Probe" ist im Sinne der Erfindung das Material zu verstehen, das den zu bestimmenden direkten Gerinnungsinhibitor vermutlich enthält. Der Begriff Probe umfasst insbesondere menschliche oder tierische Körperflüssigkeiten, vornehmlich Blut, Plasma, Serum und Urin. Sofern die Probe von einem einzigen Individuum stammt wird sie auch als Patientenprobe bezeichnet.

Die Probe kann beispielsweise einem konventionellen Gerinnungsfaktoraktivitätstest unterworfen werden, wobei der Probe eine definierte Menge desjenigen aktivierten Gerinnungsfaktors zugegeben wird, für welchen der zu bestimmende direkte Gerinnungsinhibitor spezifisch ist. Bei einem Verfahren zur Bestimmung eines Thrombininhibitors wird konsequenterweise eine definierte Menge Thrombin zugegeben; bei einem Verfahren zur Bestimmung eines Faktor Xa-Inhibitors wird eine definierte Menge Faktor Xa zugegeben, und die Inhibitor-abhängige Inaktivierung der Aktivität des zugegebenen Gerinnungsfaktors wird gemessen. Grundsätzlich können rekombinante, gentechnisch hergestellte oder aus natürlichem oder in vitro-kultiviertem Rohmaterial isolierte Gerinnungsfaktoren verwendet werden. Rohmaterialien für die Gewinnung eines Gerinnungsfaktors können z.B. tierische, beispielsweise bovine, oder humane Gewebe oder Körperflüssigkeiten (z.B. Blut, Plasma, Lymphflüssigkeit) sein, Überstände oder Lysate tierischer oder humaner Zellkulturen oder Kulturen eukaryontischer Zellen oder von Mikroorganismen, wie Bakterien oder Pilzen, die einen rekombinante Gerinnungsfaktor exprimieren. Die notwendigen Schritte zur Aktivierung inaktiver Gerinnungsfaktoren sind dem Fachmann hinlänglich bekannt.

Bevorzugterweise wird die Inhibitor-abhängige Inaktivierung der amidolytischen Aktivität des zugegebenen Gerinnungsfaktors mit Hilfe eines chromogenen, fluorogenen, amperogenen oder anderweitig markierten Substrats ermittelt, das spezifisch von dem aktivierten Gerinnungsfaktor gespalten wird. Unter einem "Substrat mit Spezifität für einen Gerinnungsfaktor" ist ein Substrat zu verstehen, das hinreichend spezifisch von einem proteolytischen Gerinnungsfaktor umgesetzt wird, wobei bei der spezifischen Substratumsetzung ein nachweisbares Spaltprodukt freigesetzt wird. Insbesondere spaltbare Substrate, welche mindestens eine Spaltstelle für einen aktivierten Gerinnungsfaktor aufweisen, sind dem Fachmann hinlänglich bekannt. Ein spaltbares Substrat kann ein synthetisch, rekombinant oder biotechnologisch hergestelltes Molekül oder ein natürliches Molekül sein, das durch Einwirken des aktivierten proteolytischen Gerinnungsfaktors in zwei Spaltprodukte zerlegt wird. Ein spaltbares Substrat kann ganz oder teilweise aus einem Peptid bestehen. Bevorzugterweise umfasst es zumindest im Bereich der Spaltstelle einen Peptidanteil.

Bei der abspaltbaren Signalgruppe ("Label") eines Substrats kann es sich z.B. um einen im sichtbaren Bereich des Spektrums bestimmbaren Farbstoff, einen Fluoreszenzfarbstoff oder einen im UV-Bereich bestimmbaren Farbstoff handeln. Beispielsweise werden Peptide verwendet, die an der Carboxygruppe eines Argininrests einen durch Amidbindung gebundenen Farbstoffrest aufweisen. Besonders geeignet sind hierfür p-Nitroanilidgruppen (pNA) und 5-Amino-2-Nitro-Benzoesäurederivate (ANBA), sowie von diesen durch Substitution abgeleitete Farbstoffe, welche nach Abspaltung vom Peptidanteil durch eine photometrische Messung bei 405 nm Wellenlänge quantifiziert werden können. Bevorzugterweise besteht der Peptidanteil eines spaltbaren Substrats aus 3 bis etwa 150 Aminosäureresten. In den Patentdokumenten EP-A1-0034122 und US 4,508,644 sind eine Vielzahl von geeigneten chromogenen Peptidsubstraten, deren Herstellung und deren Verwendung in gerinnungsdiagnostischen Tests beschrieben, z.B. zur Bestimmung der Gerinnungsfaktoren Thrombin und Xa.

Die Menge des freigesetzten signalbildenden Spaltprodukts, z.B. der chromogenen, fluorogenen oder amperogenen Gruppe, verhält sich umgekehrt proportional zur Inhibitoraktivität bzw. -konzentration in der Probe. Mit Hilfe einer Kalibrationskurve, erstellt aus der Messung von Kalibratorlösungen mit bekannten Aktivitäten eines therapeutischen Inhibitors, lässt sich die Menge des Inhibitors in einer Patientenprobe korrekt quantifizieren.

Die amidolytische Aktivität kann kinetisch oder als Endpunktbestimmung ausgewertet werden. Bei der kinetischen Methode wird die Reaktion, d.h. die verbliebene Gerinnungsfaktoraktivität als Maß für die vorhandene Inhibitoraktivität, anhand der Umsatzrate des spaltbaren Substrats quantifiziert. Bei der Endpunktbestimmung wird nach einer vorbestimmten Messzeit die Spaltreaktion gestoppt und die Menge des freigesetzten Spaltprodukts gemessen.

Die folgenden Beispiele dienen der Veranschaulichung und sind nicht als Einschränkung zu verstehen.

### BEISPIELE

### BEISPIEL 1: Herstellung universaler Kontrollmaterialien für die Bestimmung eines Thrombin- und eines Faktor Xa-Inhibitors

Es sollten universale Kontrollmaterialien bereitgestellt werden, die gleichermaßen zur Qualitätskontrolle eines Tests zur quantitativen Bestimmung von Dabigatran (therapeutischer Thrombininhibitor) und eines Tests zur quantitativen Bestimmung von Rivaroxaban (therapeutischer Faktor Xa-Inhibitor) verwendbar sind.

Bei dem Test zur quantitativen Bestimmung von Dabigatran wird in einem automatischen Analysegerät die Probe mit einem Reagenz I enthaltend eine definierte Thrombinmenge und mit einem Reagenz II enthaltend eine definierte Menge eines durch Thrombin hydrolysierbaren Substrates (Tos-Gly-Pro-Arg-ANBA-Isopropylamid) vermischt, und es wird die Geschwindigkeit des Anstiegs der Absorption des Reaktionsansatzes bei 405 nm gemessen, welche ein Maß für die Substratspaltung durch Thrombin darstellt. Die absoluten Messwerte werden mit einer zuvor in demselben Messsystem (Reagenzien und Analysegerät) bestimmten Kalibrationskurve verglichen und so die Dabigatran-Konzentration (ng/mL) bestimmt. Die Kalibrationskurve war zuvor mit Kalibratorplasmen mit bekannter Dabigatran-Konzentration erstellt worden.

Bei dem Test zur quantitativen Bestimmung von Rivaroxaban wird in einem automatischen Analysegerät die Probe mit einem Reagenz I enthaltend eine definierte Faktor Xa-Menge und mit einem Reagenz II enthaltend eine definierte Menge eines durch Faktor Xa hydrolysierbaren Substrates (Suc-Ile-Glu(piperidin-1-yl)-Gly-Arg-pNA) vermischt, und es wird die Geschwindigkeit des Anstiegs der Absorption des Reaktionsansatzes bei 405 nm gemessen, welche ein Maß für die Substratspaltung durch Faktor Xa darstellt. Die absoluten Messwerte werden mit einer zuvor in demselben Messsystem (Reagenzien und Analysegerät) bestimmten Kalibrationskurve verglichen und so die Rivaroxaban-Konzentration (ng/mL) bestimmt. Die Kalibrationskurve war zuvor mit Kalibratorplasmen mit bekannter Rivaroxaban-Konzentration erstellt worden.

Durch Lösen des unspezifischen Serinproteaseinhibitors Benzamidin oder alternativ des unspezifischen Serinproteaseinhibitors 4-(2-Aminoethyl)benzensulfonylfluorid (AEBSF, Pefabloc SC^{®}, Roche Diagnostics GmbH, Mannheim, Deutschland) in HEPES/BSA-Puffer-Lösung (0,05 M HEPES, 10 g/L BSA, pH-Wert 7,4) wurden Kontrolllösungen mit unterschiedlicher Benzamidinkonzentration (im Bereich zwischen 3 mM und 100 mM) oder alternativ mit unterschiedlicher AEBSF-Konzentration (im Bereich zwischen 5 mg/mL und 50 mg/mL) hergestellt.

Einzelnen Kontrolllösungen wurden jeweils Zielwerte für die Konzentration von Dabigatran und Rivaroxaban zugewiesen, indem jeweils mehrfach ein Aliquot jeder Kontrolllösung als Probe in den oben beschriebenen Dabigatran- und Rivaroxaban-Testen analysiert wurde und die Messergebnisse mit zuvor mit Kalibratorplasmen mit bekannter Dabigatran- bzw. Rivaroxaban-Konzentration erstellten Kalibrationskurven verglichen wurden.

Die so ermittelte Dabigatran- bzw. Rivaroxaban-Konzentration, die dann der jeweiligen Kontrolllösung als Zielwerte zugeordnet wurden und die davon abgeleiteten Zielwertbereiche (in Klammern) für die ausgewählten Kontrolllösungen sind in Tabelle 1 (Benzamidin-Kontrolllösungen) und Tabelle 2 (AEBSF-Kontrolllösungen) zusammengestellt.

**Tabelle 1: Zielwerte ausgewählter erfindungsgemäßer Benzamidin-Kontrolllösungen**

| | Benzamidin 1,8 mg/mL (15 mM) | Benzamidin 1,2 mg/mL (10 mM) |
|---|---|---|
| Dabigatran (ng/mL) | 37 (30 - 44) | 25 (20 - 30) |
| Rivaroxaban (ng/mL) | 127 (102 - 152) | 102 (82 - 122) |

**Tabelle 2: Zielwerte ausgewählter erfindungsgemäßer AEBSF-Kontrolllösungen**

| | AEBSF 25 mg/mL (104 mM) | AEBSF 10 mg/mL (42 mM) |
|---|---|---|
| Dabigatran (ng/mL) | 28 (22 - 34) | 14 (11 - 17) |
| Rivaroxaban (ng/mL) | 12 (10 - 14) | 5 (4 - 6) |

Wird in einem Messsystem in den Kontrolllösungen eine Dabigatran- bzw. Rivaroxaban-Konzentration bestimmt, die dem deklarierten Zielwert entspricht bzw. innerhalb des Zielwertbereiches liegt, zeigt dies die Richtigkeit des in dem Messsystem durchgeführten Verfahrens zur quantitativen Bestimmung des therapeutischen Gerinnungsfaktorinhibitors an. Wird hingegen in den Kontrolllösungen eine Dabigatran- bzw. Rivaroxaban-Konzentration bestimmt, die von dem deklarierten Zielwert abweicht bzw. außerhalb des Zielwertbereiches liegt, zeigt dies an, dass die Richtigkeit des in dem Messsystem durchgeführten Verfahrens zur quantitativen Bestimmung des therapeutischen Gerinnungsfaktorinhibitors nicht gegeben ist. Im letzteren Fall sollten Ergebnisse, die für Patientenproben ermittelt wurden, nicht für diagnostische Zwecke verwendet werden.

## Patentansprüche

1. Verfahren zur Herstellung eines Kontrollmaterials, das frei ist von therapeutischen Inhibitoren von Gerinnungsfaktoren aus der Gruppe der Serinproteasen und das zur Verwendung in einem Verfahren zur Kontrolle der Richtigkeit von Verfahren zur quantitativen Bestimmung von therapeutischen Inhibitoren von Gerinnungsfaktoren aus der Gruppe der Serinproteasen geeignet ist, das Verfahren zur Herstellung des Kontrollmaterials umfassend die Schritte:
(a) Lösen einer definierten Menge mindestens eines unspezifischen Serinproteaseinhibitors ausgewählt aus der Gruppe Benzamidin, Phenylmethylsulfonylfluorid und 4-(2-Aminoethyl)benzensulfonylfluorid in einer wässrigen Pufferlösung, und
(b) Zuweisen eines ersten Zielwertes für die Konzentration eines ersten therapeutischen Gerinnungsfaktorinhibitors,
wobei das Zuweisen des ersten Zielwertes für die Konzentration des ersten therapeutischen Gerinnungsfaktorinhibitors wie folgt durchgeführt wird:
i) Vermischen einer ersten Teilmenge der in Schritt a) hergestellten Lösung mit einer definierten Menge eines ersten Gerinnungsfaktors und mit einem Substrat mit Spezifität für diesen Gerinnungsfaktor zu einem ersten Reaktionsansatz,
ii) Messen der Menge eines Spaltprodukts des Substrats in dem ersten Reaktionsansatz,
iii) Vergleichen der in dem ersten Reaktionsansatz gemessenen Menge des Spaltprodukts mit vorbestimmten Kalibrationswerten, die die in dem ersten Reaktionsansatz gemessene Menge des Spaltprodukts einer bekannten Konzentration des ersten therapeutischen Gerinnungsfaktorinhibitors zuordnen, und Ermitteln der zu der gemessenen Menge des Spaltprodukts dazugehörigen Konzentration des ersten therapeutischen Gerinnungsfaktorinhibitors, und
iv) Festlegen der so ermittelten Konzentration des ersten therapeutischen Gerinnungsfaktorinhibitors als ersten Zielwert des Kontrollmaterials,
wobei der erste therapeutische Gerinnungsfaktorinhibitor spezifisch den ersten Gerinnungsfaktor inhibiert und wobei der unspezifische Serinproteaseinhibitor den ersten Gerinnungsfaktor und mindestens einen weiteren Gerinnungsfaktor inhibiert.

2. Verfahren gemäß Anspruch 1, wobei das Verfahren zur Herstellung des Kontrollmaterials ferner folgende Schritte umfasst:
(c) Zuweisen eines zweiten Zielwertes für die Konzentration eines zweiten therapeutischen Gerinnungsfaktorinhibitors,
wobei das Zuweisen des zweiten Zielwertes für die Konzentration des zweiten therapeutischen Gerinnungsfaktorinhibitors wie folgt durchgeführt wird:
i) Vermischen einer zweiten Teilmenge der in Schritt a) hergestellten Lösung mit einer definierten Menge eines zweiten Gerinnungsfaktors und mit einem Substrat mit Spezifität für diesen Gerinnungsfaktor zu einem zweiten Reaktionsansatz,
ii) Messen der Menge eines Spaltprodukts des Substrats in dem zweiten Reaktionsansatz,
iii) Vergleichen der in dem zweiten Reaktionsansatz gemessenen Menge des Spaltprodukts mit vorbestimmten Kalibrationswerten, die die in dem zweiten Reaktionsansatz gemessene Menge des Spaltprodukts einer bekannten Konzentration des zweiten therapeutischen Gerinnungsfaktorinhibitors zuordnen, und Ermitteln der zu der gemessenen Menge des Spaltprodukts dazugehörigen Konzentration des zweiten therapeutischen Gerinnungsfaktorinhibitors, und
iv) Festlegen der so ermittelten Konzentration des zweiten therapeutischen Gerinnungsfaktorinhibitors als zweiten Zielwert des Kontrollmaterials,
wobei der zweite therapeutische Gerinnungsfaktorinhibitor spezifisch den zweiten Gerinnungsfaktor inhibiert.

3. Verfahren gemäß einem der vorhergehenden Ansprüche, wobei der erste therapeutische Gerinnungsfaktorinhibitor und der zweite therapeutische Gerinnungsfaktorinhibitor ausgewählt sind aus der Gruppe Thrombininhibitor und Faktor Xa-Inhibitor.

4. Verfahren gemäß Anspruch 3, wobei der Thrombininhibitor ausgewählt ist aus der Gruppe Hirudin, Orgaran, Argatroban, Dabigatran, Melagatran, Ximelagatran, Bivalirudin, Lepirudin, MCC-977, SSR-182289, TGN-255, TGN-167, ARC-183 und Odiparcil.

5. Verfahren gemäß Anspruch 3, wobei der FXa-Inhibitor ausgewählt ist aus der Gruppe Apixaban, Rivaroxaban, Edoxaban, Fondaparinux, Otamixaban, LY 517717, YM 153, DU-176b, DX-9065a und KFA-1982.

6. Verwendung eines Kontrollmaterials herstellbar gemäß einem der Ansprüche 1-5, in einem Verfahren zur Kontrolle der Richtigkeit eines Verfahrens zur quantitativen Bestimmung eines ersten therapeutischen Inhibitors eines Gerinnungsfaktors aus der Gruppe der Serinproteasen in einer Probe, wobei der erste therapeutische Gerinnungsfaktorinhibitor spezifisch einen ersten Gerinnungsfaktor inhibiert.

7. Verwendung eines Kontrollmaterials,
• das frei ist von therapeutischen Inhibitoren von Gerinnungsfaktoren aus der Gruppe der Serinproteasen, wobei ein therapeutischer Gerinnungsfaktorinhibitor ein Inhibitor ist, der spezifisch einen einzigen Gerinnungsfaktor inhibiert,
• das eine definierte Menge eines unspezifischen Serinproteaseinhibitors enthält, wobei der unspezifische Serinproteaseinhibitor einen ersten Gerinnungsfaktor und mindestens einen weiteren Gerinnungsfaktor inhibiert und ausgewählt ist aus der Gruppe Benzamidin, Phenylmethylsulfonylfluorid und 4-(2-Aminoethyl)benzensulfonylfluorid und
• dem mindestens ein erster Zielwert für die Konzentration eines ersten therapeutischen Gerinnungsfaktorinhibitors zugewiesen ist,
in einem Verfahren zur Kontrolle der Richtigkeit eines Verfahrens zur quantitativen Bestimmung des ersten therapeutischen Inhibitors eines Gerinnungsfaktors aus der Gruppe der Serinproteasen in einer Probe.

8. Verwendung eines Kontrollmaterials,
• das frei ist von therapeutischen Inhibitoren von Gerinnungsfaktoren aus der Gruppe der Serinproteasen, wobei ein therapeutischer Gerinnungsfaktorinhibitor ein Inhibitor ist, der spezifisch einen einzigen Gerinnungsfaktor inhibiert,
• das eine definierte Menge eines unspezifischen Serinproteaseinhibitors enthält, wobei der unspezifische Serinproteaseinhibitor einen ersten und mindestens einen zweiten Gerinnungsfaktor inhibiert und ausgewählt ist aus der Gruppe Benzamidin, Phenylmethylsulfonylfluorid und 4-(2-Aminoethyl)benzensulfonylfluorid und
• dem mindestens ein erster Zielwert für die Konzentration eines ersten therapeutischen Gerinnungsfaktorinhibitors und ein zweiter Zielwert für die Konzentration eines zweiten therapeutischen Gerinnungsfaktorinhibitors zugewiesen sind,
in einem ersten Verfahren zur Kontrolle der Richtigkeit eines Verfahrens zur quantitativen Bestimmung des ersten therapeutischen Inhibitors eines Gerinnungsfaktors aus der Gruppe der Serinproteasen in einer Probe und/oder in einem zweiten Verfahren zur Kontrolle der Richtigkeit eines Verfahrens zur quantitativen Bestimmung des zweiten therapeutischen Inhibitors eines Gerinnungsfaktors aus der Gruppe der Serinproteasen in einer Probe.

9. Verfahren zur Kontrolle der Richtigkeit eines Verfahrens zur quantitativen Bestimmung eines therapeutischen Inhibitors eines ersten Gerinnungsfaktors aus der Gruppe der Serinproteasen in einer Patientenprobe, wobei das Verfahren zur quantitativen Bestimmung des therapeutischen Gerinnungsfaktorinhibitors folgende Schritte umfasst:
a. Vermischen der Patientenprobe mit einer definierten Menge des ersten Gerinnungsfaktors und mit einem Substrat mit Spezifität für diesen Gerinnungsfaktor zu einem Reaktionsansatz,
b. Messen der Menge eines Spaltprodukts des Substrates in dem Reaktionsansatz,
c. Vermischen von mindestens zwei Kalibratorproben, die jeweils eine definierte Menge des therapeutischen Gerinnungsfaktorinhibitors enthalten, mit derselben definierten Menge des ersten Gerinnungsfaktors und mit demselben Substrat mit Spezifität für diesen Gerinnungsfaktor zu jeweils einem Kalibratorreaktionsansatz,
d. Messen der Menge desselben Spaltprodukts des Substrates in den Kalibratorreaktionsansätzen und Erstellen von Kalibrationswerten, die die in den Kalibratorreaktionsansätzen gemessene Menge des Spaltprodukts einer bekannten Konzentration des ersten therapeutischen Gerinnungsfaktorinhibitors zuordnen, und Ermitteln der zu der gemessenen Menge des Spaltprodukts dazugehörigen Konzentration des ersten therapeutischen Gerinnungsfaktorinhibitors in der Patientenprobe,
e. Bestimmen der Menge des in der Patientenprobe enthaltenen therapeutischen Gerinnungsfaktorinhibitors durch Vergleichen der in dem Reaktionsansatz gemessenen Menge des Spaltprodukts mit den Kalibrationswerten,
und wobei zur Kontrolle der Richtigkeit des Verfahrens zur quantitativen Bestimmung des therapeutischen Gerinnungsfaktorinhibitors zusätzliche folgende Schritte durchgeführt werden:
i) Vermischen einer Kontrollprobe bestehend aus einer Teilmenge eines gemäß Anspruch 1 hergestellten Kontrollmaterials mit derselben definierten Menge des ersten Gerinnungsfaktors und mit demselben Substrat mit Spezifität für diesen Gerinnungsfaktor zu einem Kontrollreaktionsansatz,
ii) Messen der Menge desselben Spaltprodukts des Substrates in dem Kontrollreaktionsansatz,
iii) Vergleichen der in dem Kontrollreaktionsansatz gemessenen Menge des Spaltprodukts mit den Kalibrationswerten und Ermitteln der Konzentration des ersten therapeutischen Gerinnungsfaktorinhibitors in der Kontrollprobe, und
A. Feststellen, dass die Richtigkeit des Verfahrens zur quantitativen Bestimmung des therapeutischen Gerinnungsfaktorinhibitors in der Patientenprobe gegeben ist, wenn die in Schritt iii) in der Kontrollprobe ermittelte Konzentration des ersten therapeutischen Gerinnungsfaktorinhibitors dem ersten Zielwert für die Konzentration eines ersten therapeutischen Gerinnungsfaktorinhibitors der Kontrollprobe entspricht oder
B. Feststellen, dass die Richtigkeit des Verfahrens zur quantitativen Bestimmung des therapeutischen Gerinnungsfaktorinhibitors nicht gegeben ist, wenn die in Schritt iii) in der Kontrollprobe ermittelte Konzentration des ersten therapeutischen Gerinnungsfaktorinhibitors von dem ersten Zielwert für die Konzentration eines ersten therapeutischen Gerinnungsfaktorinhibitors der Kontrollprobe abweicht.

## Claims

1. Method for preparing a control material which is free of therapeutic inhibitors of coagulation factors from the group of the serine proteases and which is suitable for use in a method for checking the accuracy of methods for quantitative determination of therapeutic inhibitors of coagulation factors from the group of the serine proteases, the method for preparing the control material comprising the steps of:
(a) dissolving a defined amount of at least one unspecific serine protease inhibitor selected from the group consisting of benzamidine, phenylmethylsulfonyl fluoride and 4-(2-aminoethyl)benzenesulfonyl fluoride in an aqueous buffer solution, and
(b) assigning a first target value for the concentration of a first therapeutic coagulation factor inhibitor,
wherein the assignment of the first target value for the concentration of the first therapeutic coagulation factor inhibitor is carried out as follows:
i) mixing a first partial amount of the solution prepared in step a) with a defined amount of a first coagulation factor and with a substrate having specificity for said coagulation factor to form a first reaction,
ii) measuring the amount of a cleavage product of the substrate in the first reaction,
iii) comparing the amount of the cleavage product measured in the first reaction with predetermined calibration values which assign the amount of the cleavage product measured in the first reaction to a known concentration of the first therapeutic coagulation factor inhibitor, and ascertaining the concentration of the first therapeutic coagulation factor inhibitor that is associated with the measured amount of the cleavage product, and
iv) defining the thus ascertained concentration of the first therapeutic coagulation factor inhibitor as the first target value of the control material,
wherein the first therapeutic coagulation factor inhibitor specifically inhibits the first coagulation factor and wherein the unspecific serine protease inhibitor inhibits the first coagulation factor and at least one further coagulation inhibitor.

2. Method according to Claim 1, wherein the method for preparing the control material further comprises the following steps:
(c) assigning a second target value for the concentration of a second therapeutic coagulation factor inhibitor,
wherein the assignment of the second target value for the concentration of the second therapeutic coagulation factor inhibitor is carried out as follows:
i) mixing a second partial amount of the solution prepared in step a) with a defined amount of a second coagulation factor and with a substrate having specificity for said coagulation factor to form a second reaction,
ii) measuring the amount of a cleavage product of the substrate in the second reaction,
iii) comparing the amount of the cleavage product measured in the second reaction with predetermined calibration values which assign the amount of the cleavage product measured in the second reaction to a known concentration of the second therapeutic coagulation factor inhibitor, and ascertaining the concentration of the second therapeutic coagulation factor inhibitor that is associated with the measured amount of the cleavage product, and
iv) defining the thus ascertained concentration of the second therapeutic coagulation factor inhibitor as the second target value of the control material,
wherein the second therapeutic coagulation factor inhibitor specifically inhibits the second coagulation factor.

3. Method according to either of the preceding claims, wherein the first therapeutic coagulation factor inhibitor and the second therapeutic coagulation factor inhibitor are selected from the group consisting of thrombin inhibitor and factor Xa inhibitor.

4. Method according to Claim 3, wherein the thrombin inhibitor is selected from the group consisting of hirudin, orgaran, argatroban, dabigatran, melagatran, ximelagatran, bivalirudin, lepirudin, MCC-977, SSR-182289, TGN-255, TGN-167, ARC-183 and odiparcil.

5. Method according to Claim 3, wherein the FXa inhibitor is selected from the group consisting of apixaban, rivaroxaban, edoxaban, fondaparinux, otamixaban, LY 517717, YM 153, DU-176b, DX-9065a and KFA-1982.

6. Use of a control material preparable according to any of Claims 1-5 in a method for checking the accuracy of a method for quantitative determination of a first therapeutic inhibitor of a coagulation factor from the group of the serine proteases in a sample, wherein the first therapeutic coagulation factor inhibitor specifically inhibits a first coagulation factor.

7. Use of a control material
• which is free of therapeutic inhibitors of coagulation factors from the group of the serine proteases, wherein a therapeutic coagulation factor inhibitor is an inhibitor which specifically inhibits a single coagulation factor,
• which contains a defined amount of an unspecific serine protease inhibitor, wherein the unspecific serine protease inhibitor inhibits a first coagulation factor and at least one further coagulation factor and is selected from the group consisting of benzamidine, phenylmethylsulfonyl fluoride and 4-(2-aminoethyl)benzenesulfonyl fluoride, and
• to which at least one first target value for the concentration of a first therapeutic coagulation factor inhibitor is assigned,
in a method for checking the accuracy of a method for quantitative determination of the first therapeutic inhibitor of a coagulation factor from the group of the serine proteases in a sample.

8. Use of a control material
• which is free of therapeutic inhibitors of coagulation factors from the group of the serine proteases, wherein a therapeutic coagulation factor inhibitor is an inhibitor which specifically inhibits a single coagulation factor,
• which contains a defined amount of an unspecific serine protease inhibitor, wherein the unspecific serine protease inhibitor inhibits a first coagulation factor and at least one second coagulation factor and is selected from the group consisting of benzamidine, phenylmethylsulfonyl fluoride and 4-(2-aminoethyl)benzenesulfonyl fluoride, and
• to which at least one first target value for the concentration of a first therapeutic coagulation factor inhibitor and one second target value for the concentration of a second therapeutic coagulation factor inhibitor are assigned,
in a first method for checking the accuracy of a method for quantitative determination of the first therapeutic inhibitor of a coagulation factor from the group of the serine proteases in a sample and/or in a second method for checking the accuracy of a method for quantitative determination of the second therapeutic inhibitor of a coagulation factor from the group of the serine proteases in a sample.

9. Method for checking the accuracy of a method for quantitative determination of a therapeutic inhibitor of a first coagulation factor from the group of the serine proteases in a patient sample, wherein the method for quantitative determination of the therapeutic coagulation factor inhibitor comprises the following steps:
a. mixing the patient sample with a defined amount of the first coagulation factor and with a substrate having specificity for said coagulation factor to form a reaction,
b. measuring the amount of a cleavage product of the substrate in the reaction,
c. mixing at least two calibrator samples, each of which contains a defined amount of the therapeutic coagulation factor inhibitor, with the same defined amount of the first coagulation factor and with the same substrate having specificity for said coagulation factor to form a calibrator reaction in each case,
d. measuring the amount of the same cleavage product of the substrate in the calibrator reactions and creating calibration values which assign the amount of the cleavage product measured in the calibrator reactions to a known concentration of the first therapeutic coagulation factor inhibitor, and ascertaining the concentration of the first therapeutic coagulation factor inhibitor in the patient sample that is associated with the measured amount of the cleavage product,
e. determining the amount of the therapeutic coagulation factor inhibitor contained in the patient sample by comparing the amount of the cleavage product measured in the reaction with the calibration values,
and wherein the additional following steps are carried out in order to check the accuracy of the method for quantitative determination of the therapeutic coagulation factor inhibitor:
i) mixing a control sample consisting of a partial amount of a control material prepared according to Claim 1 with the same defined amount of the first coagulation factor and with the same substrate having specificity for said coagulation factor to form a control reaction,
ii) measuring the amount of the same cleavage product of the substrate in the control reaction,
iii) comparing the amount of the cleavage product measured in the control reaction with the calibration values and ascertaining the concentration of the first therapeutic coagulation factor inhibitor in the control sample, and
A. establishing that the method for quantitative determination of the therapeutic coagulation factor inhibitor in the patient sample is accurate if the concentration of the first therapeutic coagulation factor inhibitor ascertained in the control sample in step iii) corresponds to the first target value for the concentration of a first therapeutic coagulation factor inhibitor of the control sample, or
B. establishing that the method for quantitative determination of the therapeutic coagulation factor inhibitor is not accurate if the concentration of the first therapeutic coagulation factor inhibitor ascertained in the control sample in step iii) deviates from the first target value for the concentration of a first therapeutic coagulation factor inhibitor of the control sample.

## Revendications

1. Procédé de préparation d'une matière de contrôle, qui est exempte d'inhibiteur thérapeutique de facteurs de coagulation du groupe des sérineprotéases et qui est propre à être utilisée dans un procédé de contrôle qu'un procédé pour la détermination quantitative d'inhibiteurs thérapeutiques de facteurs de coagulation du groupe des sérineprotéases est judicieux, le procédé de préparation de la matière de contrôle comprenant les stades :
(a) dissolution d'une quantité définie d'au moins un inhibiteur de sérineprotéase non spécifié choisi dans le groupe de la benzamidine, du fluorure de phénylméthylsulfonyle et du fluorure de 4-(2-aminoéthyl) benzènesulfonyle dans une solution aqueuse tampon, et
(b) affectation d'une première valeur cible pour la concentration d'un premier inhibiteur thérapeutique de facteur de coagulation,
dans lequel on effectue l'affectation de la première valeur cible pour la concentration du premier inhibiteur thérapeutique de facteur de coagulation de la manière suivante :
i) mélange d'un première quantité partielle de la solution préparée au stade a) à une quantité définie d'un premier facteur de coagulation et à un substrat ayant de la spécificité pour ce facteur de coagulation pour obtenir une première masse réactionnelle,
ii) mesure de la quantité d'un produit de scission du substrat dans la première masse réactionnelle,
iii) comparaison de la quantité du produit de scission, mesurée dans la première masse réactionnelle, à des valeurs d'étalonnage définies à l'avance, qui associent la quantité du produit de scission, mesurée dans la première masse réactionnelle, à une concentration connue du premier inhibiteur thérapeutique de facteur de coagulation, et détermination de la concentration, appartenant à la quantité mesurée du produit de scission, du premier inhibiteur thérapeutique de facteur de coagulation, et
iv) fixation de la concentration ainsi déterminée du premier inhibiteur thérapeutique de facteur de coagulation comme première valeur cible de la matière de contrôle,
dans lequel le premier inhibiteur thérapeutique de facteur de coagulation inhibe spécifiquement le premier facteur de coagulation et dans lequel l'inhibiteur sérineprotéase non spécifique inhibe le premier facteur de coagulation et au moins un autre facteur de coagulation .

2. Procédé suivant la revendication 1, dans lequel le procédé de préparation de la matière de contrôle comprend en outre les stades suivants :
(c) affectation d'une deuxième valeur cible pour la concentration d'un deuxième inhibiteur thérapeutique de facteur de coagulation,
dans lequel on effectue l'affectation de la deuxième valeur cible pour la concentration du deuxième inhibiteur thérapeutique de facteur de coagulation, de la manière suivante :
i) mélange d'une deuxième quantité partielle de la solution préparée au stade a) à une quantité définie d'un deuxième facteur de coagulation et à un substrat ayant de la spécificité pour ce facteur de coagulation pour obtenir une deuxième masse réactionnelle,
ii) mesure de la quantité d'un produit de scission du substrat dans la deuxième masse réactionnelle,
iii) comparaison de la quantité du produit de scission mesurée dans la deuxième masse réactionnelle à des valeurs d'étalonnage définies à l'avance, qui associent la quantité du produit de scission mesurée dans la deuxième masse réactionnelle à une concentration connue du deuxième inhibiteur thérapeutique de facteur de coagulation et détermination de la concentration, qui appartient à la quantité mesurée du produit de scission, du deuxième inhibiteur thérapeutique de facteur de coagulation, et
iv) fixation de la concentration ainsi déterminée du deuxième inhibiteur thérapeutique de facteur de coagulation comme deuxième valeur cible de la matière de contrôle,
dans lequel le deuxième inhibiteur thérapeutique de facteur de coagulation inhibe spécifiquement le deuxième facteur de coagulation.

3. Procédé suivant l'une des revendications précédentes, dans lequel le premier inhibiteur thérapeutique de facteur de coagulation et le deuxième inhibiteur thérapeutique de facteur de coagulation sont choisis dans le groupe inhibiteur de trombine et inhibiteur de facteur Xa.

4. Procédé suivant la revendication 3, dans lequel l'inhibiteur de trombine est choisi dans le groupe hirudine, orgaran, argatroban, dabigatran, melagatran, ximelagatran, bivalirudine, lepirudine, MCC-977, SSR-182289, TGN-255, TGN-167, ARC-183 et Odiparcil.

5. Procédé suivant la revendication 3, dans lequel l'inhibiteur de FXa est choisi dans le groupe apixaban, rivaroxaban, edoxaban, fondaparinux, otamixaban, LY 517717, YM 153, DU-176b, DX-9065a et KFA-1982.

6. Utilisation d'une matière de contrôle pouvant être préparée suivant l'une des revendications 1 à 5, dans un procédé de contrôle qu'un procédé de détermination quantitative d'un premier inhibiteur thérapeutique d'un facteur de coagulation du groupe des sérineprotéases dans un échantillon est judicieux, dans laquelle le premier inhibiteur thérapeutique de facteur de coagulation inhibe spécifiquement un premier facteur de coagulation.

7. Utilisation d'une matière de contrôle,
• qui est exempte d'inhibiteur thérapeutique de facteur de coagulation du groupe des sérineprotéases, dans lequel un inhibiteur thérapeutique de facteur de coagulation est un inhibiteur, qui inhibe spécifiquement un facteur de coagulation unique,
• qui contient une quantité définie d'un inhibiteur non spécifique de sérineprotéase, dans laquelle l'inhibiteur non spécifique de sérineprotéase inhibe un premier facteur de coagulation et au moins un autre facteur de coagulation et est choisi de le groupe de la benzamidine, du fluorure de phénylméthylsulfonyle et du fluorure de 4-(2-aminoéthyl) benzènesulfonyle, et
• est affectée à au moins une première valeur cible pour la concentration d'un premier inhibiteur thérapeutique de facteurs de coagulation,
dans un procédé de contrôle qu'un procédé de détermination quantitative du premier inhibiteur thérapeutique d'un facteur de coagulation du groupe des sérineprotéases dans un échantillon est judicieux.

8. Utilisation d'une matière de contrôle,
• qui est exempte d'inhibiteur thérapeutique de facteur de coagulation du groupe des sérineprotéases, dans lequel un inhibiteur thérapeutique de facteur de coagulation est un inhibiteur qui inhibe spécifiquement un facteur de coagulation unique,
• qui contient une quantité définie d'un inhibiteur non spécifique de sérine protéase, dans lequel l'inhibiteur non spécifique de sérineprotéase inhibe un premier et au moins un deuxième facteur de coagulation et est choisi dans le groupe de la benzamidine, du fluorure de phénylméthylsulfonyle et du fluorure de 4-(2-aminoéthyl) benzènesulfonyle, et
• à laquelle sont affectées au moins une première valeur cible pour la concentration d'un premier inhibiteur thérapeutique de facteur de coagulation et une deuxième valeur cible de la concentration d'un deuxième inhibiteur thérapeutique de facteur de coagulation,
dans un premier procédé de contrôle qu'un procédé de détermination quantitative du premier inhibiteur thérapeutique d'un facteur de coagulation du groupe des sérineprotéases dans un échantillon et/ou dans un deuxième procédé de contrôle qu'un procédé de détermination quantitative du deuxième inhibiteur thérapeutique d'un facteur de coagulation du groupe des sérineprotéases dans un échantillon est judicieux.

9. Procédé de contrôle qu'un procédé de détermination quantitative d'un inhibiteur thérapeutique d'un premier facteur de coagulation du groupe des sérineprotéases dans un échantillon de patient est judicieux, dans lequel le procédé de détermination quantitative de l'inhibiteur thérapeutique de facteur de coagulation comprend les stades suivants :
a. mélange de l'échantillon du patient à une quantité définie du premier facteur de coagulation et à un substrat ayant de la spécificité pour ce facteur de coagulation pour obtenir une masse réactionnelle,
b. mesure de la quantité d'un produit de scission de substrat dans la masse réactionnelle,
c. mélange d'au moins deux échantillons d'étalonnage, qui contiennent chacun une quantité définie de l'inhibiteur thérapeutique de facteur de coagulation, à la même quantité définie du premier facteur de coagulation et au même substrat ayant de la spécificité pour ce facteur de coagulation, pour obtenir respectivement une masse réactionnelle d'étalonnage,
d. mesure de la quantité du même produit de scission du substrat dans les masses réactionnelles d'étalonnage et établissement de valeurs d'étalonnage, qui associent la quantité mesurée, dans les masses réactionnelles d'étalonnage, du produit de scission, à une concentration connue du premier inhibiteur thérapeutique de facteur de coagulation et détermination de la concentration, appartenant à la quantité mesurée du produit de scission, du premier inhibiteur thérapeutique de facteur de coagulation dans l'échantillon du patient,
e. détermination de la quantité de l'inhibiteur thérapeutique de facteur de coagulation contenu dans l'échantillon du patient par comparaison de la quantité mesurée dans la masse réactionnelle, du produit de scission aux valeurs d'étalonnage,
et dans lequel, pour contrôler que le procédé de détermination quantitative de l'inhibiteur thérapeutique de facteurs de coagulation est judicieux, on effectue en outre les stades suivants :
i) mélange d'un échantillon de contrôle, constitué d'une quantité partielle d'une matière de contrôle préparée suivant la revendication 1, à la même quantité définie du premier facteur de coagulation et au même substrat ayant de la spécificité pour ce facteur de coagulation, pour obtenir une masse réactionnelle de contrôle,
ii) mesure de la quantité du même produit de scission du substrat dans la masse réactionnelle de contrôle,
iii) comparaison de la quantité, mesurée dans le mélange réactionnel de contrôle, du produit de scission aux valeurs d'étalonnage et détermination de la concentration du premier inhibiteur thérapeutique de facteur de coagulation dans l'échantillon de contrôle, et
A. constatation que le procédé de détermination quantitative de l'inhibiteur thérapeutique de facteur de coagulation dans l'échantillon du patient est judicieux, si la concentration, déterminée au stade iii) dans l'échantillon de contrôle du premier inhibiteur thérapeutique de facteur de coagulation, correspond à la première valeur cible pour la concentration d'un premier inhibiteur thérapeutique de facteur de coagulation de l'échantillon de contrôle, ou
B. constatation que le procédé de détermination quantitative de l'inhibiteur thérapeutique de facteur de coagulation n'est pas judicieux, si la concentration, déterminée au stade iii) dans l'échantillon de contrôle, du premier inhibiteur thérapeutique de facteur de coagulation s'écarte de la première valeur cible pour la concentration d'un premier inhibiteur thérapeutique de facteur de coagulation de l'échantillon de contrôle.
